# EUROPEAN PATENT APPLICATION

(11) **EP 3 795 088 A1**
(43) Date of publication of application: **24.03.2021**
(21) Application number: 20199202.1
(22) Date of filing: 12.12.2018
(51) Int. Cl.: A61B 8/00, B06B 1/06, A61B 8/12, B06B 1/02

(54) **INTRALUMINAL ULTRASOUND IMAGING DEVICE WITH SUBSTRATE SEGMENTS FOR CONTROL CIRCUITS**

(30) Priority: 12.12.2017 US 201762597655 P
(62) Divisional of application: 18826535.9
(71) Applicant: Koninklijke Philips N.V., 5656 AG Eindhoven (NL)
(72) Inventor: JACOBSL, Egbertus Reinier, 5656 AE Eindhoven (NL); WEEKAMP, Johannes Wilhelmus, 5656 AE Eindhoven (NL); HENNEKEN, Vincent Adrianus, 5656 AE Eindhoven (NL); LOUWERSE, Marcus Cornelis, 5656 AE Eindhoven (NL); DEKKER, Ronald, 5656 AE Eindhoven (NL)
(74) Representative: Philips Intellectual Property & Standards

(57) **Abstract**

An ultrasound imaging assembly (1300) is disclosed, which is configured for wrapping around a support member (600). The imaging assembly comprises a flexible substrate (1314) having a proximal region (1302), a distal region (1304), a plurality of strips in a central region (1303) stretching between and secured at their respective ends to the proximal and distal regions. Each of the plurality of strips comprises a control circuit (1314), the distal region of the flexible substrate comprises a transducer array (1310), while the proximal region comprises a connection die (1317) for electrical connection between the ultrasound imaging assembly and an image processing system. An ultrasound imaging device (102) is further disclosed, comprising the ultrasound imaging assembly.

## Description

### TECHNICAL FIELD

The present disclosure relates generally to intraluminal imaging and, in particular, to the ultrasound imaging assembly of an intraluminal imaging device. The imaging assembly can include an array of transducers positioned on a flexible substrate that is wrapped circumferentially around a support structure.

### BACKGROUND

Intravascular ultrasound (IVUS) imaging is widely used in interventional cardiology as a diagnostic tool for assessing a diseased vessel, such as an artery, within the human body to determine the need for treatment, to guide the intervention, and/or to assess its effectiveness. An IVUS device including one or more ultrasound transducers is passed into the vessel and guided to the area to be imaged. The transducers emit ultrasonic energy in order to create an image of the vessel of interest. Ultrasonic waves are partially reflected by discontinuities arising from tissue structures (such as the various layers of the vessel wall), red blood cells, and other features of interest. Echoes from the reflected waves are received by the transducer and passed along to an IVUS imaging system. The imaging system processes the received ultrasound echoes to produce a cross-sectional image of the vessel where the device is placed.

Solid-state IVUS catheters carry a sensing assembly or scanner assembly that includes an array of ultrasound transducers distributed around its circumference along with one or more integrated circuit controller chips mounted adjacent to the transducer array. The solid-state IVUS catheters are also referred to as phased array IVUS transducers or phased array IVUS devices. The controllers select individual transducer elements (or groups of elements) for transmitting an ultrasound pulse and for receiving the ultrasound echo signal. By stepping through a sequence of transmit-receive pairs, the solid-state IVUS system can synthesize the effect of a mechanically scanned ultrasound transducer but without moving parts (hence the solid-state designation). Since there is no rotating mechanical element (as in a rotational IVUS catheter), the transducer array can be placed in direct contact with the blood and vessel tissue with minimal risk of vessel trauma. Furthermore, because there is no rotating element, the electrical interface is simplified. The solid-state scanner can be wired directly to the imaging system with a simple electrical cable and a standard detachable electrical connector, rather than the complex rotating electrical interface required for a rotational IVUS device.

Manufacturing a solid-state IVUS catheter that has a small diameter to easily traverse tortuous vasculature can be challenging. In some instances, the controller chips and/or the ultrasound transducers can undesirably increase the diameter of the distal end of the catheter. In some instances, interference between the controller chips and/or the ultrasound transducers can undesirably reduce image quality.

### SUMMARY

Embodiments of the present disclosure provide improved intraluminal imaging system for generating ultrasound images within a body lumen such as a blood vessel. In that regard, the present disclosure provides for an imaging assembly including a flexible substrate and a support member around which the flexible substrate is wrapped. A transducer array is integrated into the flexible substrate, and a plurality of control circuits are disposed on the flexible substrate. For example, a proximal portion of the flexible substrate can include multiple substrate ribbons. A control circuit can be positioned on a respective substrate ribbon. The ribbon allows the outer profile of the control circuits to not extend beyond the outer profile of the transducer array. Advantageously, this configuration allows a reduced diameter for the solid-state catheter. The disclosed structural arrangement of the flexible substrate and the support member advantageously position the control circuits outside a transmission zone of the transducer array thereby reducing interference with the transmitted ultrasonic signals resulting from their contact with highly reflective control circuits. Accordingly, the disclosed embodiments can improve image resolution and quality.

In one embodiment, an intraluminal imaging device is disclosed. The intraluminal imaging device comprises a flexible elongate member configured to be inserted into a body lumen of a patient. The flexible elongate member comprises a proximal portion and a distal portion. The intraluminal imaging device further comprises an ultrasound imaging assembly disposed at the distal portion of the flexible elongate member. The ultrasound imaging assembly comprises a support member, a flexible substrate positioned around the support member and including a proximal region and a distal region, the proximal region comprising a plurality of cutouts defining a plurality of substrate ribbons, a plurality of transducer elements integrated in the distal region of the flexible substrate, and a plurality of control circuits disposed on the proximal region of the flexible substrate.

In some embodiments, the support member comprises a proximal portion and a distal portion, the proximal portion comprising a plurality of planar surfaces sized and shaped to receive a control circuit thereupon. In some embodiments, the proximal portion of the support member is hexagonal in shape. In some embodiments, the distal portion of the support member comprises a spool configured to receive the plurality of transducer elements. In some embodiments, the distal region of the flexible substrate is cylindrical in shape when positioned around the support member. In some embodiments, the flexible substrate comprises a central region that is polygonal in shape when positioned around the support member. In some embodiments, the distal, central, and proximal regions of the flexible substrate each have a different shape when positioned around the support member. In some embodiments, the proximal and central regions of the flexible substrate each comprise a plurality of cuts arranged to facilitate their respective shapes when wrapped around the support member. In some embodiments, the flexible substrate comprises a central region that comprises a plurality of collinear cuts. In some embodiments, each of the plurality of control circuits is disposed on a respective one of the plurality of substrate ribbons.

In one embodiment, a method is disclosed. The method comprises providing a flexible substrate comprising a proximal region and a distal region, the proximal region comprising a plurality of cutouts defining a plurality of substrate ribbons, the distal region including a plurality of ultrasound transducer elements integrated therein. The method further comprises locating a plurality of control circuits on the proximal region of the flexible substrate, and positioning the flexible substrate around a support member.

In some embodiments, the support member comprises a proximal portion and a distal portion, the proximal portion comprising a plurality of planar surfaces sized and shaped to receive a control circuit thereupon. In some embodiments, the proximal portion of the support member is hexagonal in shape. In some embodiments, the distal portion of the support member comprises a spool sized and shaped to receive the plurality of ultrasound transducer elements. In some embodiments, the distal region of the flexible substrate is cylindrical in shape when positioned around the support member. In some embodiments, providing the flexible substrate comprises providing a plurality of cuts in a central region of the flexible substrate such that the central region of the flexible substrate is polygonal in shape when positioned around the support member. In some embodiments, providing the flexible substrate comprises providing a plurality of collinear cuts in a central region of the flexible substrate. In some embodiments, locating the plurality of control circuits on the proximal region of the flexible substrate comprises locating the each of the plurality of control circuits on respective ones of the plurality of substrate ribbons. In some embodiments, the plurality of ultrasound transducer elements comprises a plurality of capacitive micromachined ultrasound transducers. In some embodiments, the flexible substrate connects a plurality of base substrate islands.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
Fig. 1 is a diagrammatic schematic view of an intraluminal imaging system, according to aspects of the present disclosure.
Fig. 2 is a diagrammatic top view of a portion of a flexible assembly in a flat configuration, according to aspects of the present disclosure.
Fig. 3 is a diagrammatic side view of an imaging assembly, including a transducer array in a rolled configuration around a support member, according to aspects of the present disclosure.
Fig. 4 is a diagrammatic cross-sectional side view of a distal portion of an intraluminal imaging device, according to aspects of the present disclosure.
Fig. 5 is a diagrammatic top view of a flexible assembly with control chips, according to aspects of the present disclosure.
Fig. 6A is a diagrammatic perspective view of a support member, according to aspects of the present disclosure.
Fig. 6B is a diagrammatic perspective view of a support member, according to aspects of the present disclosure.
Fig. 7A is a diagrammatic perspective view of a support member, according to aspects of the present disclosure.
Fig. 7B is a diagrammatic perspective view of a support member, according to aspects of the present disclosure.
Fig. 7C is a diagrammatic cross-sectional view of a support member, according to aspects of the present disclosure.
Fig. 8A is a diagrammatic perspective view of an imaging assembly, according to aspects of the present disclosure.
Fig. 8B is a diagrammatic cross-sectional view of an imaging assembly, according to aspects of the present disclosure.
Fig. 9 is a diagrammatic perspective view of an intraluminal imaging device, according to aspects of the present disclosure.
Fig. 10 is a flow chart of a method, according to aspects of the present disclosure.
Figs. 11 and 12 illustrate exemplary transducers arranged on an exemplary flexible substrate according to aspects of the present disclosure. In particular, Fig. 11 is a diagrammatic side view of the exemplary transducers with the flexible substrate in a flat configuration, and Fig. 12 is a diagrammatic side view of the exemplary transducers with the flexible substrate in a curved (or rolled) configuration.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

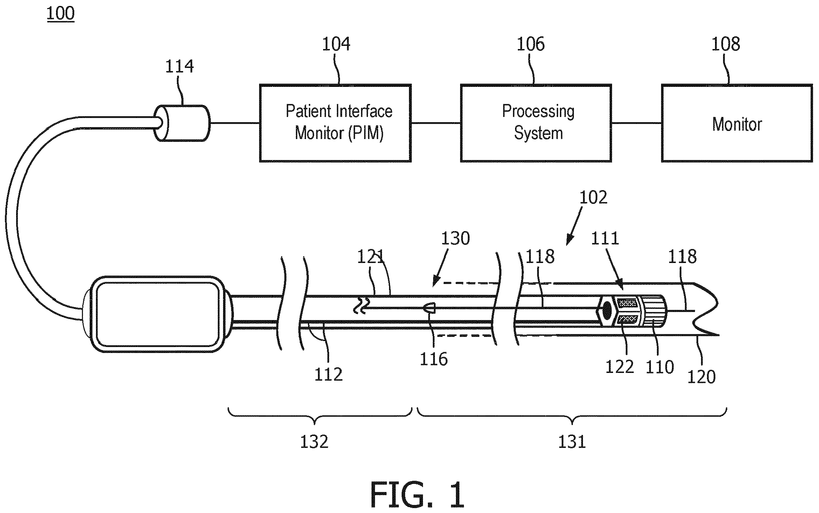

Fig. 1 is a diagrammatic schematic view of an intraluminal imaging system 100, according to aspects of the present disclosure. The intraluminal imaging system 100 may include an intraluminal imaging device 102, a patient interface module (PIM) 104, a processing system 106, and a monitor 108.

In some embodiments, the intraluminal imaging device 102 may comprise an ultrasound imaging device, e.g., an intravascular ultrasound (IVUS) imaging device, sized and shaped to be positioned within an anatomy of a patient. In that regard, the intraluminal imaging device 102 may obtain ultrasound imaging data from within the patient's anatomy. Generally, the intraluminal imaging device 102 may comprise a catheter, a guide wire, guide catheter, or combinations thereof. The intraluminal imaging device 102 may comprise a flexible elongate member 121. As used herein, "elongate member" or "flexible elongate member" includes at least any thin, long, flexible structure structurally arranged (e.g., sized and/or shaped) to be positioned within a lumen (or body lumen) of a patient's anatomy. As shown in FIG. 1, the intraluminal imaging device 102 is positioned within a body lumen 120. In some cases, the body lumen 120 is a blood vessel. In some embodiments, the flexible elongate member 121 may include one or more layers of braided metallic and/or polymer strands or a flexible hypotube. The braided layer(s) can be tightly or loosely braided in any suitable configuration, including any suitable per in count (pic). In some embodiments, the flexible elongate member 121 can include one or more metallic and/or polymer coils. All or a portion of the flexible elongate member 121 may have any suitable geometric cross-sectional profile (*e.g.*, circular, oval, rectangular, square, elliptical, etc.) or non-geometric cross-sectional profile. For example, the flexible elongate member 121 can have a generally cylindrical profile with a circular cross-sectional profile that defines an outer diameter of the flexible elongate member 121. For example, the outer diameter of the flexible elongate member 121 can be any suitable value for positioning within a patient's anatomy, including between approximately 1 French (Fr) and approximately 15 Fr, including values such as 1 Fr, 2 Fr, 2.4 Fr, 2.5 Fr, 3 Fr, 3.5 Fr, 5 Fr, 7 Fr, 8.2 Fr, 9 Fr, and/or other suitable values both larger and smaller. In that regard, the intraluminal imaging device 102 may have an outer diameter less than 3 Fr. In particular, the intraluminal imaging device 102 may have an outer diameter of .014 inches, and outer diameter of .016 inches, or an outer diameter in therebetween.

The intraluminal imaging device 102 may include one or more lumens extending along all or a portion of the length of the flexible elongate member 121. Said lumens may be sized and shaped to receive and/or guide one or more diagnostic or therapeutic instruments through the patient's anatomy. In that regard, FIG. 1 illustrates guidewire 118 extending through a lumen of the intraluminal imaging device 102 between an exit/entry port 116 and a distal end of the intraluminal imaging device 102. The exit/entry port 116 is disposed near a junction 130 at which a distal portion 131 is coupled to a proximal portion 132. Accordingly, in some instances the intraluminal imaging device 102 may be a rapid-exchange catheter.

The intraluminal imaging device 102 may include an imaging assembly 111 mounted at the distal portion 131 near a distal end of the intraluminal imaging device 102. The imaging assembly 111 can include a transducer array 110 comprising a plurality of transducer elements. The intraluminal imaging device 102 may emit ultrasonic energy from the transducer array 110. The ultrasonic energy is reflected by tissue structures, e.g., walls of body lumen 120, surrounding the transducer array 110, and the ultrasound echo signals are received by the transducer array 110. The transducer array 110 can include any suitable number of individual transducers between 2 transducers and 1000 transducers, including values such as 2 transducers, 4 transducers, 36 transducers, 64 transducers, 128 transducers, 500 transducers, 812 transducers, and/or other values both larger and smaller. The transducer array 110 may be a phased array. The transducer array 110 may be divided into segments, e.g., one or more rows and/or columns, that may be independently controlled and activated. The transducer array 110 and/or individual transducers may be arranged to emit and/or recieve ultrasonic energy at an oblique angle relative to a longitudinal axis of the intraluminal imaging device 102.

The transducers of the transducer array 110 can be piezoelectric micromachined ultrasound transducers (PMUT), capacitive micromachined ultrasonic transducers (CMUT), single crystal, lead zirconate titanate (PZT), PZT composite, other suitable transducer types, and/or combinations thereof. Exemplary capacitive micromachined ultrasound transducers (cMUTs) are disclosed, for example, in U.S. App. No. 14/812,792, filed July 29, 2015, and titled "Intravascular Ultrasound Imaging Apparatus, Interface Architecture, and Method of Manufacturing," which is hereby incorporated by reference in its entirety. Depending on the transducer material, the manufacturing process for the transducer(s) can include dicing, kerfing, grinding, sputtering, wafer technologies (e.g., SMA, sacrificial layer deposition), other suitable processes, and/or combinations thereof.

For diagnosis and/or imaging, the center frequency of the transducer array 110 can be between 10 MHz and 70 MHz, for example, including values such as 10 MHz, 20 MHz, 30 MHz, 40 MHz, 45 MHz, 60 MHz, and/or other suitable values both larger and smaller. For example, lower frequencies (e.g., 10 MHz, 20 MHz) can advantageously penetrate further into the anatomy 102, such that more of a patient's anatomy is visible in the ultrasound images. Higher frequencies (e.g., 45 MHz, 60 MHz) can be better suited to generate more detailed ultrasound images of the patient's anatomy and/or fluid within the body lumen 120. In some embodiments, the frequency of the ultrasonic energy emitted by the transducer array 110 is tunable. In some instances, the transducer array 110 can be tuned to receive wavelengths associated with the center frequency and/or one or more harmonics of the center frequency. In some instances, the frequency of the emitted ultrasonic energy can be modified by the voltage of the applied electrical signal and/or the application of a biasing voltage to the transducer array 110.

The imaging assembly 111 can further include one or more control circuits 122. In various contexts, control circuits 122 may be controllers, control chips, application specific integrated circuits (ASIC), or combinations thereof. Control circuits 122 may be configured to select particular transducer elements to be used for transmission/reception of ultrasonic energy, to provide transmission trigger signals to activate transmitter circuitry to generate an electrical pulse to excite the selected transducer elements, and/or to accept amplified echo signals received from the selected transducer elements. Multiple control circuit 122 configurations with various numbers of master circuits and slave circuits can be used to create a single ultrasound wave or multi-firing ultrasound wave device.

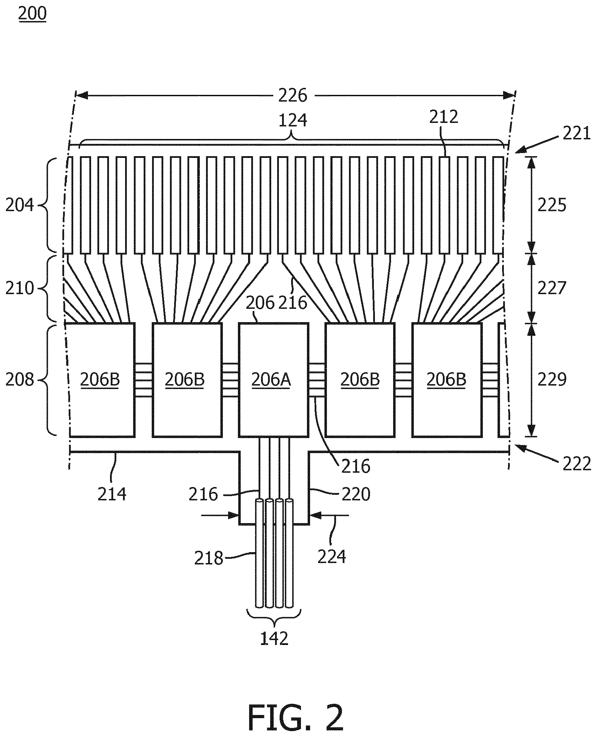

The intraluminal imaging device 102 may include one or more electrical conductors 112 extending from the proximal portion 132 to the distal portion 131. The electrical conductor 112 is a transmission line bundle including a plurality of conductors, including one, two, three, four, five, six, seven, eight, or more conductors 218 (Fig. 2). It is understood that any suitable gauge wire can be used for the conductors 218. In an embodiment, the electrical conductor 112 can include a four-conductor transmission line arrangement with, e.g., 41 American wire gauge (AWG) wires. In an embodiment, the electrical conductor 112 can include an eight-conductor transmission line arrangement utilizing, e.g., 44 AWG wires. In some embodiments, 43 AWG wires can be used. The electrical conductors 112 may carry electrical signals between the PIM 104 and/or the processing system 106 and the imaging assembly 111. The electrical conductor 112 may terminate in a PIM connector 114. The PIM connector 114 may electrically couple the electrical conductor 112 to the PIM 104 and may further physically couple the intraluminal imaging device 102 to the PIM 104.

The PIM 104 may transfer received echo signals to the processing system 106 where an ultrasound image (including, in some cases, flow information) may be reconstructed and displayed on the monitor 108. In that regard, the PIM 104 facilitates communication of signals between the processing system 106 and the transducer array 110. This communication of signals may include the steps of: (1) providing commands to control circuits 122 to select the particular transducer element to be used to transmit and receive ultrasonic energy, (2) providing the transmit trigger signals to the control circuits 122 to activate the transmitter circuitry to generate an electrical pulse to excite the selected transducer elements, and/or (3) accepting amplified echo signals received from the selected transducer array elements via amplifiers included on the control circuits 122. In some embodiments, the PIM 104 performs preliminary processing of the echo data prior to relaying the data to the processing system 106. In examples of such embodiments, the PIM 104 performs amplification, filtering, and/or aggregating of the data. In an embodiment, the PIM 104 also supplies high- and low-voltage direct current (DC) power to support operation of the intraluminal imaging device 102, including circuitry within the transducer array 110.

The intraluminal imaging device 102 may be used to examine any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves, chambers, or other parts of the heart, and/or other systems of the body. In addition to natural structures, the intraluminal imaging device 102 may be used to examine man-made structures such as, but without limitation, heart valves, stents, shunts, filters and other devices.

In various embodiments, the intraluminal imaging device 102 can obtain imaging data associated with intravascular ultrasound (IVUS) imaging, forward looking intravascular ultrasound (FL-IVUS) imaging, intravascular photoacoustic (IVPA) imaging, intracardiac echocardiography (ICE), forward-looking ICE (FLICE), transesophageal echocardiography (TEE), and/or other suitable imaging modalities. The intraluminal imaging device may also be configured to obtain physiologic data associated with pressure, flow, temperature, a fractional flow reserve (FFR) determination, a functional measurement determination, a coronary flow reserve (CFR) determination, optical coherence tomography (OCT), computed tomography, intravascular palpography, and/or other types of physiologic data. In some embodiments, the intraluminal imaging device 102 includes one or more features similar to traditional solid-state IVUS catheters, such as the EagleEye® catheter available from Volcano Corporation and those disclosed in U.S. Patent No. 7,846,101 hereby incorporated by reference in its entirety.

Fig. 2 is a diagrammatic top view of a portion of a flexible assembly 200, according to aspects of the present disclosure. The flexible assembly 200 includes a transducer array 124 formed in a transducer region 204 and transducer control logic dies 206 (including dies 206A and 206B) formed in a control region 208, with a transition region 210 disposed therebetween. The transducer array 124 includes an array of ultrasound transducers 212. The transducer control logic dies 206 are mounted on a flexible substrate 214 into which the transducers 212 have been previously integrated. The flexible substrate 214 is shown in a flat configuration in Fig. 2. Though six control logic dies 206 are shown in Fig. 2, any number of control logic dies 206 may be used. For example, one, two, three, four, five, six, seven, eight, nine, ten, or more control logic dies 206 may be used.

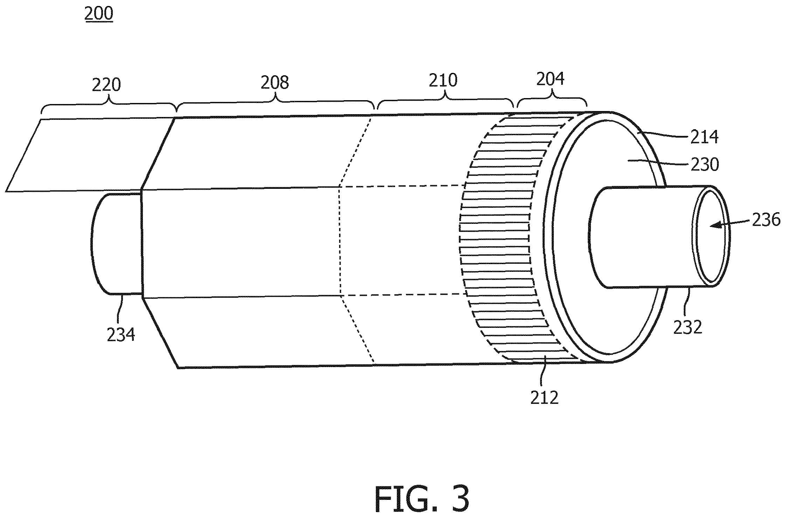

The flexible substrate 214, on which the transducer control logic dies 206 and the transducers 212 are mounted, provides structural support and interconnects for electrical coupling. The flexible substrate 214 may be constructed to include a film layer of a flexible polyimide material such as KAPTON™ (trademark of DuPont). Other suitable materials include polyester films, polyimide films, polyethylene napthalate films, or polyetherimide films, liquid crystal polymer, other flexible printed semiconductor substrates as well as products such as Upilex® (registered trademark of Ube Industries) and TEFLON® (registered trademark of E.I. du Pont). In the flat configuration illustrated in Fig. 2, the flexible substrate 214 has a generally rectangular shape. As shown and described herein, the flexible substrate 214 is configured to be wrapped around a support member 230 (Fig. 3) in some instances. Therefore, the thickness of the film layer of the flexible substrate 214 is generally related to the degree of curvature in the final assembled flexible assembly 110. In some embodiments, the film layer is between 5 µm and 100 µm, with some particular embodiments being between 5 µm and 25.1 µm, e.g., 6 µm.

The transducer control logic dies 206 is a non-limiting example of a control circuit. The transducer region 204 is disposed at a distal portion 221 of the flexible substrate 214. The control region 208 is disposed at a proximal portion 222 of the flexible substrate 214. The transition region 210 is disposed between the control region 208 and the transducer region 204. Dimensions of the transducer region 204, the control region 208, and the transition region 210 (e.g., lengths 225, 227, 229) can vary in different embodiments. In some embodiments, the lengths 225, 227, 229 can be substantially similar or, the length 227 of the transition region 210 may be less than lengths 225 and 229, the length 227 of the transition region 210 can be greater than lengths 225, 229 of the transducer region and controller region, respectively.

The control logic dies 206 are not necessarily homogenous. In some embodiments, a single controller is designated a master control logic die 206A and contains the communication interface for cable 142 which may serve as an electrical conductor, e.g., electrical conductor 112, between a processing system, e.g., processing system 106, and the flexible assembly 200. Accordingly, the master control circuit may include control logic that decodes control signals received over the cable 142, transmits control responses over the cable 142, amplifies echo signals, and/or transmits the echo signals over the cable 142. The remaining controllers are slave controllers 206B. The slave controllers 206B may include control logic that drives a transducer 212 to emit an ultrasonic signal and selects a transducer 212 to receive an echo. In the depicted embodiment, the master controller 206A does not directly control any transducers 212. In other embodiments, the master controller 206A drives the same number of transducers 212 as the slave controllers 206B or drives a reduced set of transducers 212 as compared to the slave controllers 206B. In an exemplary embodiment, a single master controller 206A and eight slave controllers 206B are provided with eight transducers assigned to each slave controller 206B.

To electrically interconnect the control logic dies 206 and the transducers 212, in an embodiment, the flexible substrate 214 includes conductive traces 216 formed in the film layer that carry signals between the control logic dies 206 and the transducers 212. In particular, the conductive traces 216 providing communication between the control logic dies 206 and the transducers 212 extend along the flexible substrate 214 within the transition region 210. In some instances, the conductive traces 216 can also facilitate electrical communication between the master controller 206A and the slave controllers 206B. The conductive traces 216 can also provide a set of conductive pads that contact the conductors 218 of cable 142 when the conductors 218 of the cable 142 are mechanically and electrically coupled to the flexible substrate 214. Suitable materials for the conductive traces 216 include copper, gold, aluminum, silver, tantalum, nickel, and tin, and may be deposited on the flexible substrate 214 by processes such as sputtering, plating, and etching. In an embodiment, the flexible substrate 214 includes a chromium adhesion layer. The width and thickness of the conductive traces 216 are selected to provide proper conductivity and resilience when the flexible substrate 214 is rolled. In that regard, an exemplary range for the thickness of a conductive trace 216 and/or conductive pad is between 1-5 µm. For example, in an embodiment, 5 µm conductive traces 216 are separated by 5 µm of space. The width of a conductive trace 216 on the flexible substrate may be further determined by the width of the conductor 218 to be coupled to the trace/pad.

The flexible substrate 214 can include a conductor interface 220 in some embodiments. The conductor interface 220 can be a location of the flexible substrate 214 where the conductors 218 of the cable 142 are coupled to the flexible substrate 214. For example, the bare conductors of the cable 142 are electrically coupled to the flexible substrate 214 at the conductor interface 220. The conductor interface 220 can be tab extending from the main body of flexible substrate 214. In that regard, the main body of the flexible substrate 214 can refer collectively to the transducer region 204, controller region 208, and the transition region 210. In the illustrated embodiment, the conductor interface 220 extends from the proximal portion 222 of the flexible substrate 214. In other embodiments, the conductor interface 220 is positioned at other parts of the flexible substrate 214, such as the distal portion 221, or the flexible substrate 214 may lack the conductor interface 220. A value of a dimension of the tab or conductor interface 220, such as a width 224, can be less than the value of a dimension of the main body of the flexible substrate 214, such as a width 226. In some embodiments, the substrate forming the conductor interface 220 is made of the same material(s) and/or is similarly flexible as the flexible substrate 214. In other embodiments, the conductor interface 220 is made of different materials and/or is comparatively more rigid than the flexible substrate 214. For example, the conductor interface 220 can be made of a plastic, thermoplastic, polymer, hard polymer, etc., including polyoxymethylene (e.g., DELRIN®), polyether ether ketone (PEEK), nylon, Liquid Crystal Polymer (LCP), and/or other suitable materials.

Fig. 3 illustrates a rolled configuration of the flexible substrate 214. In some instances, the flexible assembly 200 is transitioned from a flat configuration (Fig. 2) to a rolled or more cylindrical configuration (Fig. 3). For example, in some embodiments, techniques are utilized as disclosed in one or more of U.S. Patent No. 6,776,763, titled "ULTRASONIC TRANSDUCER ARRAY AND METHOD OF MANUFACTURING THE SAME" and U.S. Patent No. 7,226,417, titled "HIGH RESOLUTION INTRAVASCULAR ULTRASOUND SENSING ASSEMBLY HAVING A FLEXIBLE SUBSTRATE," each of which is hereby incorporated by reference in its entirety. Fig. 3 is a diagrammatic perspective view with the flexible substrate 214 in the rolled configuration around a support member 230, according to aspects of the present disclosure. The support member 230 can be referenced as a unibody in some instances. The support member 230 can be composed of a metallic material, such as stainless steel, or non-metallic material, such as a plastic or polymer as described in U.S. Provisional Application No. 61/985,220, "Pre-Doped Solid Substrate for Intravascular Devices," filed April 28, 2014, ('220 Application) the entirety of which is hereby incorporated by reference herein. The support member 230 can be a ferrule having a distal portion 232 and a proximal portion 234. The support member 230 can be tubular in shape and define a lumen 236 extending longitudinally therethrough. The lumen 236 can be sized and shaped to receive the guide wire 118. The support member 230 can be manufactured using any suitable process. For example, the support member 230 can be machined and/or electrochemically machined or laser milled, such as by removing material from a blank to shape the support member 230, or molded, such as by an injection molding process.

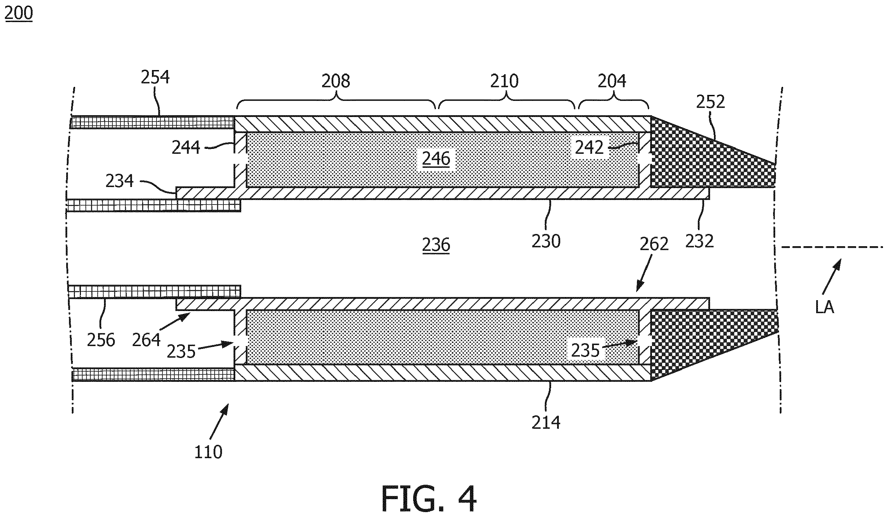

Referring now to Fig. 4, shown there is a diagrammatic cross-sectional side view of a distal portion of the intraluminal imaging device 102, including the flexible substrate 214 and the support member 230, according to aspects of the present disclosure. The support member 230 can be referenced as a unibody in some instances. The support member 230 can be composed of a metallic material, such as stainless steel, or non-metallic material, such as a plastic or polymer as described in U.S. Provisional Application No. 61/985,220, "Pre-Doped Solid Substrate for Intravascular Devices," filed April 28, 2014, the entirety of which is hereby incorporated by reference herein. The support member 230 can be ferrule having a distal portion 262 and a proximal portion 264. The support member 230 can define a lumen 236 extending along the longitudinal axis LA. The lumen 236 is in communication with the entry/exit port 116 and is sized and shaped to receive the guide wire 118 (Fig. 1). The support member 230 can be manufactured according to any suitable process. For example, the support member 230 can be machined and/or electrochemically machined or laser milled, such as by removing material from a blank to shape the support member 230, or molded, such as by an injection molding process. In some embodiments, the support member 230 may be integrally formed as a unitary structure, while in other embodiments the support member 230 may be formed of different components, such as a ferrule and stands 242, 244, that are fixedly coupled to one another. In some cases, the support member 230 and/or one or more components thereof may be completely integrated with inner member 256. In some cases, the inner member 256 and the support member 230 may be joined as one, e.g., in the case of a polymer support member.

Stands 242, 244 that extend vertically are provided at the distal and proximal portions 262, 264, respectively, of the support member 230. The stands 242, 244 elevate and support the distal and proximal portions of the flexible substrate 214. In that regard, portions of the flexible substrate 214, such as the transducer portion 204 (or transducer region 204), can be spaced from a central body portion of the support member 230 extending between the stands 242, 244. The stands 242, 244 can have the same outer diameter or different outer diameters. For example, the distal stand 242 can have a larger or smaller outer diameter than the proximal stand 244 and can also have special features for rotational alignment as well as control chip placement and connection. To improve acoustic performance, any cavities between the flexible substrate 214 and the surface of the support member 230 are filled with a backing material 246. The liquid backing material 246 can be introduced between the flexible substrate 214 and the support member 230 via passageways 235 in the stands 242, 244. In some embodiments, suction can be applied via the passageways 235 of one of the stands 242, 244, while the liquid backing material 246 is fed between the flexible substrate 214 and the support member 230 via the passageways 235 of the other of the stands 242, 244. The backing material can be cured to allow it to solidify and set. In various embodiments, the support member 230 includes more than two stands 242, 244, only one of the stands 242, 244, or neither of the stands. In that regard the support member 230 can have an increased diameter distal portion 262 and/or increased diameter proximal portion 264 that is sized and shaped to elevate and support the distal and/or proximal portions of the flexible substrate 214.

The support member 230 can be substantially cylindrical in some embodiments. Other shapes of the support member 230 are also contemplated including geometrical, non-geometrical, symmetrical, non-symmetrical, cross-sectional profiles. As the term is used herein, the shape of the support member 230 may reference a cross-sectional profile of the support member 230. Different portions the support member 230 can be variously shaped in other embodiments. For example, the proximal portion 264 can have a larger outer diameter than the outer diameters of the distal portion 262 or a central portion extending between the distal and proximal portions 262, 264. In some embodiments, an inner diameter of the support member 230 (e.g., the diameter of the lumen 236) can correspondingly increase or decrease as the outer diameter changes. In other embodiments, the inner diameter of the support member 230 remains the same despite variations in the outer diameter.

A proximal inner member 256 and a proximal outer member 254 are coupled to the proximal portion 264 of the support member 230. The proximal inner member 256 and/or the proximal outer member 254 can be part of the flexible elongate member 121 (Fig. 1). The proximal inner member 256 can be received within a proximal flange 234. The proximal outer member 254 abuts and is in contact with the flexible substrate 214. A distal member 252 is coupled to the distal portion 262 of the support member 230. For example, the distal member 252 is positioned around the distal flange 232. The distal member 252 can abut and be in contact with the flexible substrate 214 and the stand 242. The distal member 252 can be the distal-most component of the intraluminal imaging device 102.

One or more adhesives can be disposed between various components at the distal portion of the intraluminal imaging device 102. For example, one or more of the flexible substrate 214, the support member 230, the distal member 252, the proximal inner member 256, and/or the proximal outer member 254 can be coupled to one another via an adhesive.

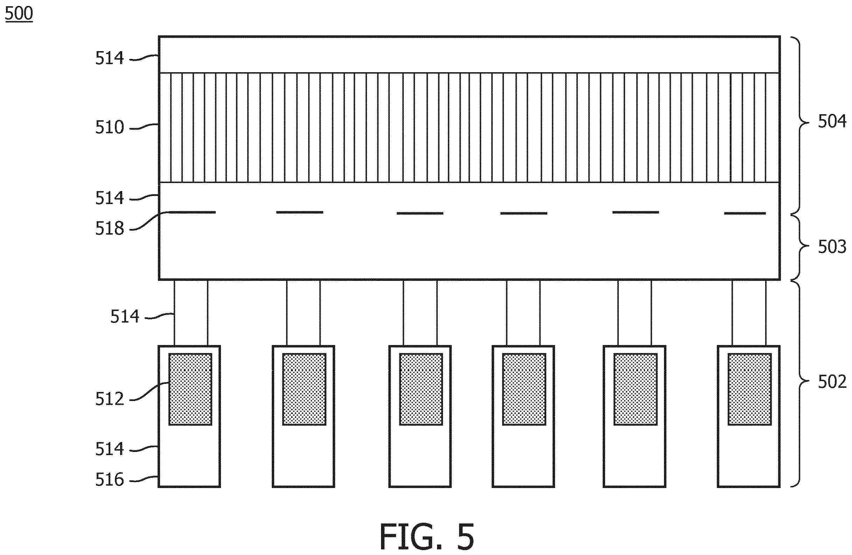

Turning now to Fig. 5, a flexible assembly 500 is described. The flexible assembly 500 may make up a portion of an imaging assembly of an intraluminal imaging device. As the term is used herein, an imaging assembly may comprise a flexible assembly wrapped around a support member. Though not shown in Fig. 5, the flexible assembly 500 may comprise a conductor providing for the transmission of electrical signals between the flexible assembly 500 and one or more elements of an intraluminal imaging system, e.g., a PIM or image processing system. Though illustrated in a flat state in Fig. 5, the flexible assembly 500 may be configured to be wrapped around a support member one, two, three, four, or more times. In that regard, the flexible assembly 500 may comprise a transducer array 510 integrated in a flexible substrate 514 and a plurality of control circuits 512 disposed on the flexible substrate 514.

The transducer array 510 and plurality of control circuits 512 may be spaced apart longitudinally from each other when wrapped around a support member and may in some circumstances be referred to as being in-line. Spacing the transducer array 510 and control circuits 512 apart longitudinally may reduce the proportion of an outer diameter, e.g., of an intraluminal imaging device, attributable to said elements advantageously leaving more room for acoustic backing material which may improve the imaging performance of the transducer array 510. For example, the acoustic backing material may be insulating in nature and may prevent or limit ultrasonic energy being transmitted toward an interior of the intraluminal imaging device and may absorb any echoes returning from the interior.

As similarly described above, the flexible substrate 514 may comprise one or more electrical traces providing for the transmission of electrical signals between the transducer array 510 and the plurality of control circuits 512. The flexible substrate 514 may itself be disposed on a base substrate 516. In that regard, the flexible substrate 514 may be disposed between the base substrate 516 and one or more of the transducer array 510 and/or the control circuits 512. For example, the flexible substrate 514 may be disposed between one of the control circuits 512 and the base substrate 516. The base substrate 516 may comprise silicon and may be flexible. In some cases, the base substrate 516 may not be a unitary structure but may instead comprise two or more separate pieces. For example, the base substrate 516 may comprise a series of strips or islands.

The transducer array 510 may comprise a plurality of CMUT transducer elements which may be formed using wafer processing techniques. As similarly described above, the transducer array 510 may be a phased array and may be under the influence of the control circuits 512. For example, the control circuits 512 may send electrical signals to the transducer array and thereby trigger the emission of ultrasonic pulses from the transducer array. In some cases, individual control circuits 512 may control individual sections of the transducer array 510. Control circuits 512 may be soldered onto the flexible substrate 514 and/or onto the base substrate 516. In some cases, formation of the flexible assembly 500 is a wafer level process. The transducer array 510 may be processed onto a base substrate, e.g., silicon wafer. After the transducer array 510 is processed onto the base substrate, the base substrate may be provided with a polyimide layer, a metal interconnect layer, and a second polyimide layer and patterned into a desired shape. Then the base substrate is etched away from the backside to define base substrate islands. The interconnect areas between the base substrate islands may be flexible as the base substrate has been etched way leaving only the polyimide and interconnect behind.

The flexible assembly 500 may comprise a distal region 504, a central region 503, and a proximal region 502. The distal region 504 may include the flexible substrate 514 with the transducer array 510 disposed thereon. The flexible substrate 514 may be disposed on the base substrate 516 in the distal region 504. The transducer array 510 may abut a distal-most edge of the flexible assembly 500 or may be spaced away from the distal-most edge. The distal region 504 of the flexible assembly 500 may be structured so as to have a cylindrical shape when wrapped around a support member. The distal region 504 may be bounded on its proximal end by a plurality of cuts 518 formed in the flexible substrate 514 and defining the distal end of the central region 503.

The central region 503 may include the flexible substrate 514, which may or may not be disposed on the base substrate 516. A plurality of cuts 518 may be formed in the flexible substrate 514. The plurality of cuts 518 may be formed in any pattern. Accordingly, the patterns discussed herein are exemplary in nature and are not intended to limit the scope of the disclosure. In some cases, the plurality of cuts 518 may include a series of colinear cuts 518. The pattern of cuts 518 in the central region 503 may be arranged to facilitate a transition in shape from the proximal region 502 to the distal region 504 when the flexible assembly 500 is wrapped around a support member. In that regard, the proximal region 502 may be polygonal in shape, e.g., hexagonal, when wrapped around a support member while the distal region 504 may be rounded in shape, e.g., cylindrical, when wrapped around the support member. Accordingly, the central region 503 may include cuts 518 arranged so as to facilitate the central region 503 adopting a polygonal shape, e.g., dodecagonal, with more vertices than that of the proximal region 502 when the flexible assembly 500 is wrapped around a support member.

The proximal region 502 may feature the flexible substrate 514 and/or base substrate 516 cut into a plurality of substrate ribbons. The substrate ribbons may feature one or more regions of increased flexibility. As such, the regions of increased flexibility may be more flexible than other regions of the flexible assembly 500. The regions of increased flexibility may be thinner, have a lesser diameter, or both, than other regions of the flexible assembly 500. For example, the regions of increased flexibility may include only one layer, e.g., only the flexible substrate 514, while other regions of the flexible assembly 500 include two or more, e.g., at least the flexible substrate 514 and the base substrate 516. The regions of increased flexibility may facilitate a transition in shape, size, or both, from the proximal region 502 to the central region 503 when the flexible assembly 500 is wrapped around a support member.

Each substrate ribbon may include one or more control circuit 512 disposed thereupon. In that regard, the control circuits 512 may be disposed on the flexible substrate 514 and/or the base substrate 516. The substrate ribbons may be arranged to facilitate the proximal region 502 adopting a polygonal shape, e.g., hexagonal, when wrapped around a support member. In that regard, the substrate ribbons may be arranged so as to correspond to faces of a polygonal support member such that control circuits 512 will be positioned on said faces when the flexible assembly 500 is wrapped around the support member. As the term is used herein, faces of the support member may be planar surfaces connecting two vertices. The polygonal shape of the proximal region 502 may advantageously reduce an outer profile of the control circuits 512 disposed on the substrate ribbons. In some cases, the outer profile of the control circuits 512 does not extend beyond an outer profile of the transducer array 510.

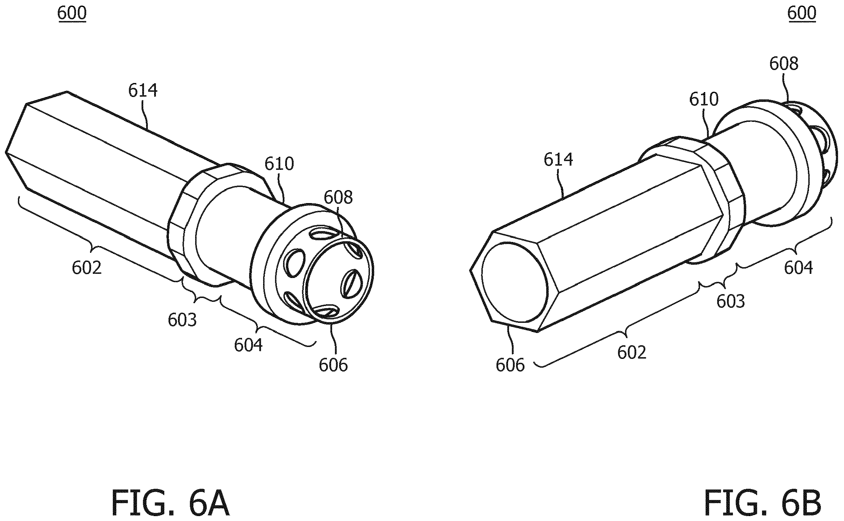

Turning now to Figs. 6A and 6B, a support member 600 is described. The support member 600 may have a proximal region 602, a central region 603, and a distal region 604 as well as a lumen 606 extending therethrough. The support member 600 may be made of stainless steel, a polymer, or another suitable material and may shield a transducer array from electrical impulses emanating from a guide wire or other tool extending through the lumen 606. The support member 600 may reinforce an imaging assembly of an intraluminal imaging device, e.g., by supporting a flexible assembly. In that regard, the support member 600 may be sized and shaped to receive a flexible assembly, e.g., flexible assembly 500, wrapped therearound.

The distal region 604 of the support member 600 may comprise one or more apertures 608. The apertures 608 may be disposed about a longitudinal axis extending from a proximal end to a distal end of the support member 600. In some cases, the apertures 608 may facilitate attachment of one or more elements of an intraluminal imaging device to the support member 600. For example, a distal tip element of an intraluminal imaging device may be anchored to the apertures 608. The apertures 608 may be circular, ovular, elliptical, square, rectangular, triangular, some other shape, or combinations thereof. The distal region 604 of the support member 600 may additionally comprise a spool 610. The spool 610 may be sized and shaped to receive a transducer array of a flexible assembly wrapped therearound. A cylindrical, central area of the spool 610 may be bounded on each end by rims of increased diameter. For example, the distal end of the spool 610 may feature a round or cylindrical rim while the proximal end of the spool 610 may feature a polygonal, e.g., dodecagonal, rim which defines the central region 603 of the support member 600. A transducer array may be wrapped around and supported by the rims while resultant empty space between the cylindrical, central area and the transducer array is filled with acoustic backing material configured to acoustically insulate the transducer array from echoes coming from the center of an intraluminal imaging device in which the transducer array is implemented.

As described above, the central region 603 may comprise a polygonal rim. The polygonal rim may include three, four, five, six, seven, eight, nine, ten, eleven, twelve, or more vertices and a corresponding number of faces. The polygonal rim may be structured to support a transducer array and/or a central region of a flexible assembly. In some cases, the polygonal rim may be structured to give shape to a portion, e.g., a central region, of a flexible assembly wrapped around the support member 600 and may be structured to facilitate a transition in shape in the flexible assembly from polygonal to cylindrical as the flexible assembly extends from the proximal region 602 to the distal region 604. In that regard, the polygonal rim may feature more vertices and faces than a body 614 of the proximal region 602.

Though not shown, the proximal region 602 of the support member 600 may comprise one or more apertures 608 which may be disposed about the longitudinal axis of the support member 600 and may facilitate attachment of one or more elements of an intraluminal imaging device to the support member 600. For example, a flexible elongate member of an intraluminal imaging device may be anchored to the apertures 608 of the proximal region 602. The proximal region 602 may comprise a body 614. The body 614 may be polygonal in shape, e.g., hexagonal, and may include a plurality of faces sized and shaped to receive a control circuit thereon. In an embodiment, the faces of the body 614 are structured so that an outer profile of control circuits disposed thereon does not extend beyond that of a transducer array wrapped around the spool 610. In that regard, the outer diameter of the body 614 may be less than that of the rims of the spool 610.

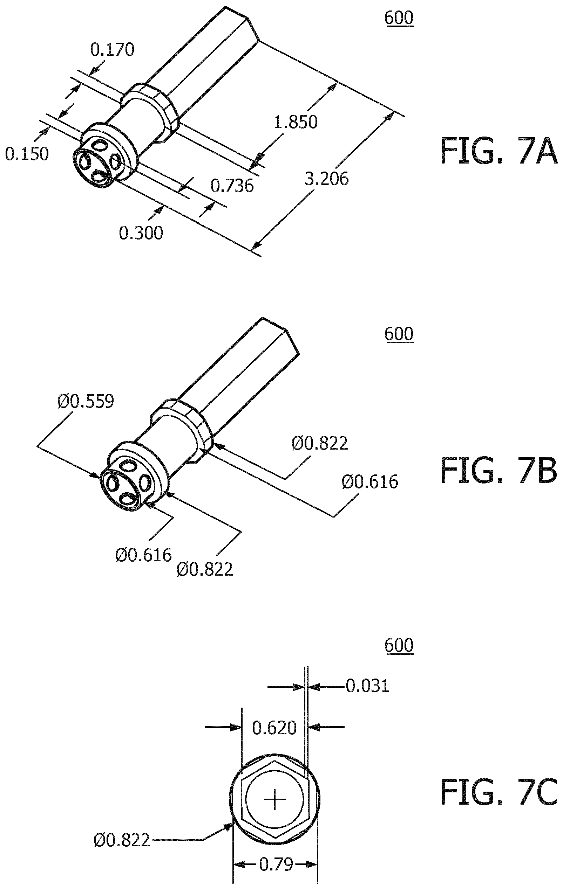

Figs. 7A-7C illustrate exemplary dimensions for the support member 600 measured in millimeters. In that regard, Fig. 7A illustrates the length of various elements of the support member 600, Fig. 7B illustrates the diameter of various elements of the support member 600, and Fig. 7C illustrates miscellaneous measurements of various elements of the support member 600.

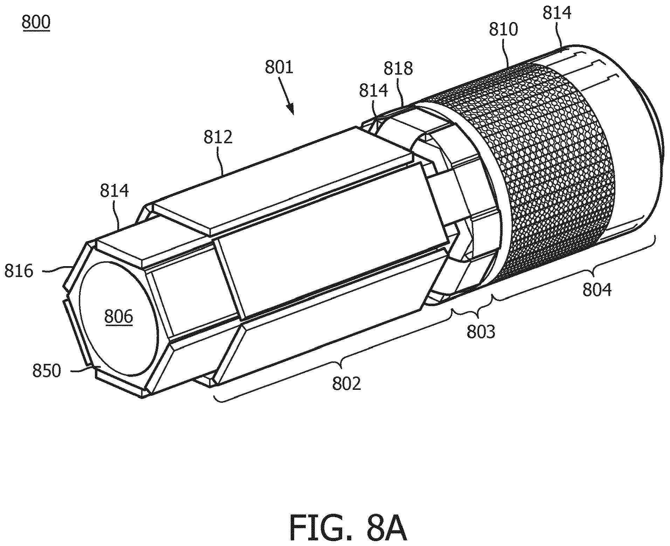

Turning now to Fig. 8A, an imaging assembly 800 is described. The imaging assembly 800 may be a component of an intraluminal imaging device. The imaging assembly 800 comprises a flexible assembly 801 wrapped around a support member 850 with a lumen 806 extending therethrough. As similarly described above, the flexible assembly 801 comprises a transducer array 810 integrated in a flexible substrate 814 and a plurality of control circuits 812 disposed on the flexible substrate 814 which may itself be disposed on a base substrate 816. Some portions of the flexible substrate 814 can be disposed on the base substrate 816, while other portions only include the flexible substrate 814. The portions including only the flexible substrate 814 may have relatively more flexibility in some embodiments. The flexible substrate 801 features a plurality of cuts 818. The imaging assembly 800 comprises a proximal region 802, a central region 803, and a distal region 804 which may align with proximal, central, and distal regions of the flexible assembly 801 and support member 850. In that regard, the imaging assembly 800 may transition from a hexagonal shape at its proximal region 802, to a dodecagonal shape at its central region 803, to a cylindrical shape at its distal region 804. In some cases, the proximal region 802 and/or the central region 803 may be cylindrical in shape. An outer profile of the transducer array 810 may be equal to or greater than an outer profile of the control circuits 812. The slits 818 may advantageously enable the control regions proximal region 802 to be depressed toward the support member. In this manner, the outer profile/diameter of the proximal region 802 (including the control circuits 812) can be equal to or less than the outer profile/diameter of the distal region 804 (including the transducer array 810).

The proximal region 802 may be referenced as a depressible region that advantageously allow the control circuits 812 to be depressed toward the support member thereby reducing an outer profile or outer diameter of the control circuits 812. In some cases, depression of the control circuits 812 toward the support member may reduce the outer profile of the control circuits 812 to such a degree that the outer profile of the control circuits 812 does not extend beyond an outer profile of the transducer array 810.

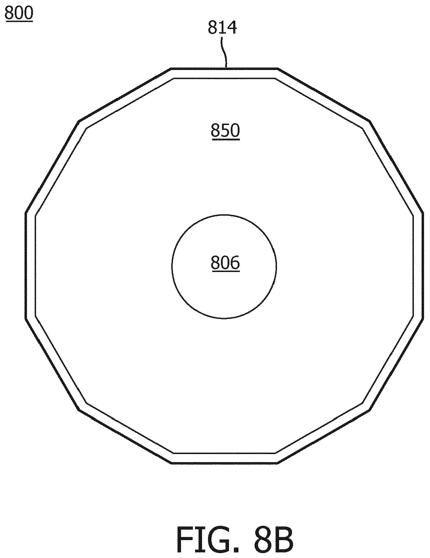

Fig. 8B is a diagrammatic, cross-sectional view of the imaging assembly 800 at the central region 803. In that regard, the imaging assembly 800 may comprise the flexible substrate 814 wrapped around the support member 850 with the lumen 806 extending therethrough. The support member 850 may have a substantially cylindrical shape with a plurality of flat faces. For example, the support member 850 may be octagonal, decagonal, dodecagonal, or some other shape. In some cases, the shape of the support member 850 and/or slits in the flexible substrate 814 may facilitate a change in shape from the distal region 804 to the proximal region 802. For example, the distal region 804 may be cylindrical while the central region 803 is dodecagonal and the proximal region 802 is hexagonal.

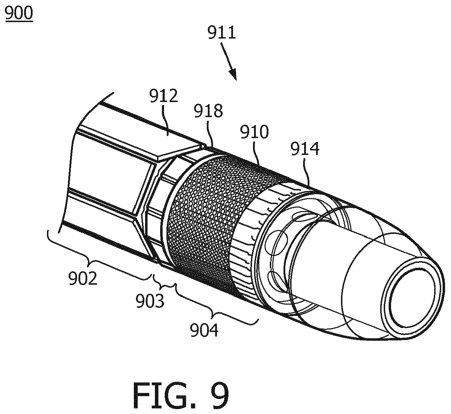

Turning now to Fig. 9, an intraluminal imaging device 900 is described. Fig. 9 illustrates a distal portion of the intraluminal imaging device 900. As similarly described above, the intraluminal imaging device 900 may be sized and shaped for introduction into a body lumen, e.g., a blood vessel, of a patient's anatomy and may be configured to perform one or more imaging operations including intravascular ultrasound (IVUS) imaging, forward looking intravascular ultrasound (FL-IVUS) imaging, intravascular photoacoustic (IVPA) imaging, intracardiac echocardiography (ICE), forward-looking ICE (FLICE), transesophageal echocardiography (TEE), and/or other suitable imaging modalities.

The intraluminal imaging device 900 may comprise an imaging assembly 911. The imaging assembly 911 may comprise a flexible assembly including a transducer array 910 and a plurality of control circuits 912 disposed on a flexible substrate 914 featuring a plurality of cuts 918. The imaging assembly 911 may further comprise a support member around which the flexible assembly is wrapped. The transducer assembly 910 may be aligned or colocated longitudinally with a spool of the support member and the plurality of control circuits 812 may be located on faces of a body of the support member. In that regard, proximal regions 902, central regions 903, and distal regions 904 of the flexible assembly and support member may be co-located longitudinally in the assembled intraluminal imaging device 900.

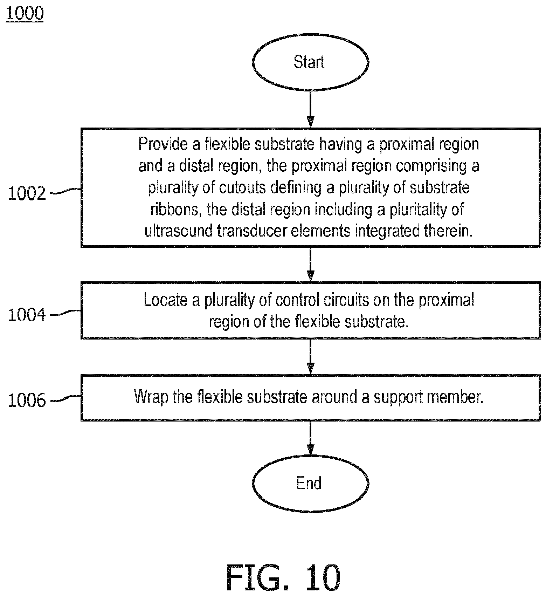

Turning now to Fig. 10, a method 1000 is described. The method 1000 may be implemented to assemble an intraluminal imaging device and/or imaging assembly such as those described herein. The method 1000 begins at block 1002 where a flexible substrate is provided. The flexible substrate has a proximal region and a distal region. The proximal region comprises a plurality of cutouts defining a plurality of substrate ribbons. The distal region includes a plurality of ultrasound transducer elements integrated therein. Providing the flexible substrate may comprise providing a plurality of cuts in a central region of the flexible substrate such that a central region of the flexible substrate is polygonal in shape when wrapped around a support member. Providing the flexible substrate may comprise providing a series of collinear cuts in a central region of the flexible substrate. In some cases, providing the flexible substrate may comprise providing a plurality of cuts between a central region of the flexible substrate and the distal region of the flexible substrate. At block 1004, a plurality of control circuits is located on the proximal region of the flexible substrate. In some cases, locating the plurality of control circuits on the proximal region of the flexible substrate comprises locating the each of the plurality of control circuits on respective ones of the plurality of substrate ribbons. The flexible substrate is wrapped around a support member at block 1006.

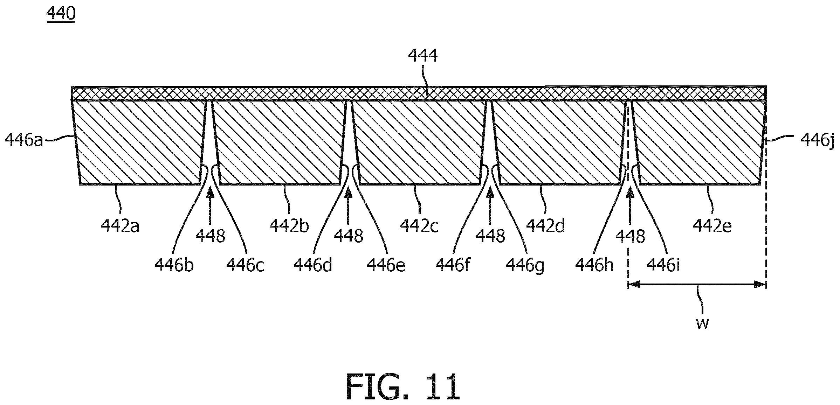

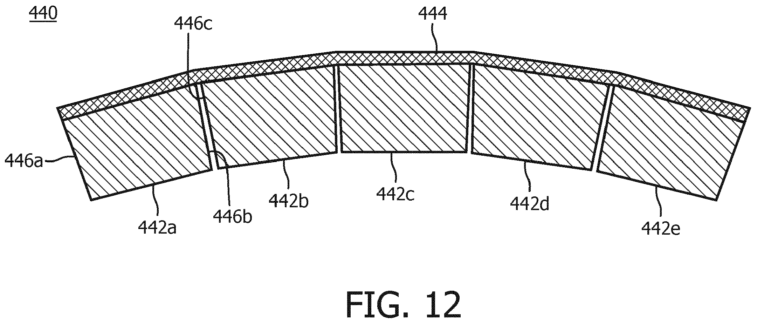

Figs. 11 and 12 illustrate an array 440 of transducer elements 442 arranged on a substrate 444 according to aspects of the present disclosure. In particular, Fig. 11 is a diagrammatic side view of the array 440 of transducer elements 442a-e with the substrate 444 in a flat configuration, and Fig. 12 is a diagrammatic side view of the array 440 of transducer elements 442a-e with the substrate 444 in a curved (or rolled) configuration. As shown in Fig. 11, the transducer elements 442a-e are arranged linearly on the substrate 444. In some embodiments, the substrate 444 comprises a flexible substrate. The transducer elements 442 include a width W. The width W may range from 20 to 100 microns. The transducer elements 442a-e include angled sidewalls 446a-j. The sidewalls 446 are non-perpendicular to one another, thereby defining wedge-shaped trenches 448 between the non-perpendicular sidewalls 446 and therefor facilitate bending. In some examples, the sidewalls 446 can be angled approximately between 1° and 45°, between 1° and 30°, between 1° and 15°, between 1° and 10°, between 1° and 5°, including values such as 22.5°, 11.25°, 9°, 5.625°, 4.5°, 2.8125°, and/or other suitable values, both larger and smaller. The angle of the sidewalls 446 can be based on the number of transducer elements 442, the diameter of the scanner assembly 110, the diameter of the imaging device 102, the dimensions of the transducer elements 442, the spacing between adjacent transducer elements 442, etc. In some embodiments, the sidewalls 446 of all transducer elements can be angled by the same amount. In other embodiments, the sidewalls 446 of different transducers elements are angled by different amounts.

As shown in Fig. 12, when the substrate 444 is curved or flexed, the transducer elements 442 contact one another along the entire length of their sidewalls. For example, the sidewall 446b of the transducer element 442a comes into full contact with the sidewall 446c of the transducer element 442b. Thus, this non-perpendicular trench configuration maximizes the surface area available on the substrate for the transducer elements 442. Other non-perpendicular separations of the transducer elements 442 are contemplated. For example, in some embodiments, the sidewalls 446 may be curved or serpentine, where neighboring sidewalls 446 are configured to rest against one another or contact one another along at least a portion of the length of the trench 448 when the flexible substrate 444 is flexed or in a curved configuration. One method of manufacture may be anisotropic dry etching or an appropriate combination of anisotropic dry etching and isotropic dry etching, such that the desired trench sidewall profile is obtained.

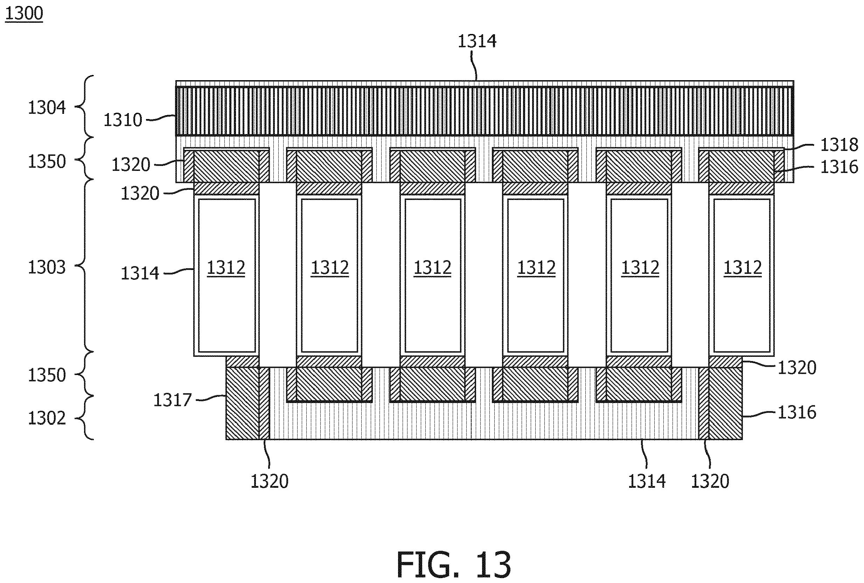

Turning now to Fig. 13, a flexible assembly 1300 is described. The flexible assembly 1300 may make up a portion of an imaging assembly of an intraluminal imaging device. The flexible assembly 1300 may comprise a silicon region 1317 leading to a connection die for connecting one or more conductor providing for the transmission of electrical signals between the flexible assembly 1300 and one or more elements of an intraluminal imaging system, e.g., a PIM or image processing system. Though illustrated in a flat state in Fig. 13, the flexible assembly 1300 may be configured to be transitioned into a cylindrical or cylindrical toroid configuration. For example, the flexible assembly 1300 can be wrapped around a support member one, two, three, four, or more times. In that regard, the flexible assembly 1300 may comprise a transducer array 1310 integrated in a flexible substrate 1314 and a plurality of control circuits 1312 disposed on the flexible substrate 1314.

The transducer array 1310 and plurality of control circuits 1312 may be spaced apart longitudinally from each other when wrapped around a support member and may in some circumstances be referred to as being in-line. Spacing the transducer array 1310 and control circuits 512 apart longitudinally may reduce the proportion of an outer diameter, e.g., of an intraluminal imaging device, attributable to said elements advantageously leaving more room for acoustic backing material which may improve the imaging performance of the transducer array 1310. For example, the acoustic backing material may be insulating in nature and may prevent or limit ultrasonic energy being transmitted toward an interior of the intraluminal imaging device and may absorb any echoes returning from the interior.

As similarly described above, the flexible substrate 1314 may comprise one or more electrical traces providing for the transmission of electrical signals between the transducer array 1310 and the plurality of control circuits 1312. The flexible substrate 1314 may itself be disposed on a base substrate 1316. In that regard, the flexible substrate 1314 may be disposed between the base substrate 1316 and one or more of the transducer array 1310 and/or the control circuits 1312. For example, the flexible substrate 1314 may be disposed between one of the control circuits 1312 and the base substrate 1316. The base substrate 1316 may comprise silicon and may be flexible. In some cases, the base substrate 1316 may not be a unitary structure but may instead comprise two or more separate pieces. For example, the base substrate 1316 may comprise a series of strips or islands. Such strips or islands may be connected to other portions of the flexible assembly 1300 by flexible regions 1320, which may comprise a portion of flexible substrate 1314 without base substrate 1316 backing.

In that regard, the flexible assembly may comprise a distal region 1304, a proximal region 1302, and a central region 1303. The proximal region 1302, distal region 1304, and central region 1303 may be separated by transition regions 1350. Flexible regions 1320 and/or cuts 1318 of transition regions 1350 may facilitate wrapping the flexible assembly 1300 around a support member, e.g., by facilitating a change in shape over the length of the flexible assembly 1300. For example, the distal region 1304 may adopt a cylindrical shape when wrapped around a support member while the central region 1303 adopts a hexagonal shape. The proximal region 1302 and the distal region 1304 may advantageously improve the structural integrity of the substrate strips or islands of the central region 1303 by securing said strips or islands at both ends. For example, the proximal region 1302 and the distal region 1304 can keep the strips or islands of the central region 1303 in place on a support member during assembly, thereby increasing ease of assembly. The proximal region 1302 that is closed or attached can also contribute to a more robust scanner interface to the catheter shaft.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. An ultrasound imaging assembly (1300) configured for wrapping around a support member (600), comprising:
a flexible substrate (1314) comprising a proximal region (1302), a distal region (1304), a plurality of strips in a central region (1303) stretching between and secured at their respective ends to the proximal and distal regions, wherein each of the plurality of strips comprises a control circuit (1314);
wherein the distal region of the flexible substrate comprises a transducer array (1310); and
wherein the proximal region comprises a connection die (1317) for electrical connection between the ultrasound imaging assembly and an image processing system.

2. The imaging assembly of claim 1, further comprising the support member, wherein the flexible substrate is wrapped around and secured to the support member.

3. The imaging assembly of claim 2, wherein the distal portion of the support member comprises a spool (610) configured to receive the transducer array.

4. The imaging assembly of claim 3, wherein the distal region of the flexible substrate is cylindrical in shape when wrapped around the support member.

5. The imaging assembly of any of the claims 2 to 4, wherein the support member comprises a central region with polygonal shaped cross-section, which receives the plurality of strips of the central region of the flexible substrate when wrapped around the support member.

6. The imaging assembly of any of the preceding claims, wherein the connection die comprises a silicon region (1317).

7. An ultrasound imaging device (102) comprising a flexible elongate member (121) configured to be inserted into a body lumen of a patient, wherein the ultrasound imaging assembly according to any of the claims 2 to 6 is disposed at the distal portion of the flexible elongate member.

8. An ultrasound imaging system (100) comprising an ultrasound imaging device (102) according to claim 7, a patient interface module (104), a processing system (106) and a monitor (108).

9. A method of manufacturing an ultrasound imaging assembly, the method comprising:
providing an ultrasound imaging assembly according to claim 1;
wrapping and securing the flexible substrate of the ultrasound imaging assembly around a support member.
